Europäisches Patentamt

European Patent Office

Office européen des brevets

(19)

(11) Publication number: **0 099 428**
**B1**

(12) # EUROPEAN PATENT SPECIFICATION

(45) Date of publication of patent specification: **26.11.86**

(51) Int. Cl.⁴: $A\ 61\ L\ 15/00$

(21) Application number: **82303762.7**

(22) Date of filing: **16.07.82**

(54) Absorbent web structure.

(43) Date of publication of application:
**01.02.84 Bulletin 84/05**

(45) Publication of the grant of the patent:
**26.11.86 Bulletin 86/48**

(84) Designated Contracting States:
**BE DE FR GB IT NL SE**

(56) References cited:
**GB-A-1 259 983**
**US-A-3 920 508**
**US-A-4 286 082**
**US-A-4 333 979**

(73) Proprietor: **MITSUI PETROCHEMICAL INDUSTRIES, LTD.**
**2-5, Kasumigaseki 3-chome Chiyoda-ku Tokyo 100 (JP)**

(72) Inventor: **Horimoto, Koji**
**2-2, Muronoki-machi 1-chome Iwakuni-shi Yamaguchi-ken (JP)**
Inventor: **Morita, Yoshinori**
**68-7, Muronoki-machi 4-chome Iwakuni-shi Yamaguchi-ken (JP)**

(74) Representative: **Myerscough, Philip Boyd et al J.A.Kemp & Co. 14, South Square Gray's Inn London, WC1R 5EU (GB)**

The file contains technical information submitted after the application was filed and not included in this specification

EP 0 099 428 B1

Courier Press, Leamington Spa, England.

## Description

This invention relates to an absorbent web structure which can exhibit desired mechanical strength properties and excellent absorbing properties when used as disposable diapers, sanitary napkins, medical sponges, wound-treating pads and towels.

More specifically, this invention pertains to an absorbent web structure obtained by a dry sheet forming process composed of a mixture of 5 to 50% by weight of short fibers of a thermoplastic resin rendered hydrophilic with a surface-active agent and 95 to 50% by weight of cellulosic fibers, said thermoplastic short fibers being melt-bonded to impart self-supporting property to the structure, characterized in that

(i) said thermoplastic short fibers are rendered hydrophilic by forming an aqueous slurry of the fibers containing a nonionic surface-active agent and then dehydrating the slurry, and

(ii) said nonionic surface-active agent has (a) an HLB value of from 2 to 20 and (b) a melting point equal to, or higher than, the temperature of the slurry at the time of the dehydrating treatment described in (i) above.

Japanese Laid-Open Patent Publication No. 17455/1978 discloses that a three-dimensional absorbent structure is obtained by mixing a cellulosic fibrous material such as wood pulp with fibers of a thermoplastic resin and consolidating the mixture under moderate heat and pressure, and used as disposable diapers, etc. Japanese Laid-Open Patent Publication No. 16611/1980 also discloses that a water-absorbent sheet obtained by dry sheet formation from a mixture of wood pulp, fibers of a thermoplastic resin and a powder of a water-holding polymeric material such as an acrylic acid-grafted polyglucose or saccharose polymer can be used as disposable diapers.

In these techniques, the thermoplastic fibrous material is desirably mixed as uniformly as possible in the wood pulp, and the bonding treatment under heat melts and bonds the thermoplastic fibers and anchors the wood pulp at various points. It is known that such an absorbent web structure has increased entanglement of the individual fibers and excellent shape stability such as elasticity and recovery.

If the amount of thermoplastic short fibers is large in such an absorbent web structure composed of a mixture of short fibers of thermoplastic resin and cellulosic fibers in which the thermoplastic fibers are melt-bonded to impart self-supporting property to the structure, the structure has improved mechanical strength, but cannot avoid a reduction in absorbing properties. The proportion of the thermoplastic short fibers used should therefore be determined depending upon the end uses by considering the mechanical properties and water absorbing properties of the final product.

It is known on the other hand that in order to improve the hydrophilicity of thermoplastic short fibers, their surface is treated with polyvinyl alcohol, polyacrylic acid, etc. (Japanese Patent Publication No. 47049/1977 corresponding to U.S. Patent No. 3,920,508).

When surface-active agents are spray-coated on these short fibers in order to improve their hydrophilicity, no great difference in the effect of rendering them hydrophilic is seen depending upon the types of the surfactants.

Frequently, these short fibers are handled in the form of an aqueous slurry. In particular, pulp-like short fibers (synthetic pulp) produced by a flashing method are in the form of an aqueous slurry in the final step of their production. It is desirable therefore to improve their hydrophilicity while they are in the form of an aqueous slurry.

If an attempt is made to improve the hydrophilicity of short fibers of thermoplastic resin by adding a surface-active agent to an aqueous slurry of the thermoplastic short fibers and then dehydrating the slurry, it often results in unsatisfactory hydrophilicity or no improvement of hydrophilicity is obtained.

We made investigations in order to overcome these difficultes, and newly found that the type and HLB value of the surface-active agent used, and the relation between the melting point of the surface-active agent and the temperature of the aqueous slurry at the time of dehydration predominantly affect the absorbing properties, particularly the absorbency rate, of an absorbent web structure composed of short fibers of a thermoplastic resin rendered hydrophilic with the surface-active agent and cellulosic fibers, the thermoplastic short fibers being melt-bonded to impart self-supporting property to the structure.

We further studied the relation among these factors, and have now found that an absorbent web structure having much improved absorbing properties can be provided by using short fibers of thermoplastic resin rendered hydrophilic by a surface-active agent which are characterized by the following (i) and (ii).

(i) The thermoplastic short fibers are rendered hydrophilic by first forming an aqueous slurry of the thermoplastic short fibers containing a nonionic surface-active agent and then dehydrating the slurry, and

(ii) the nonionic surface-active agent has (a) an HLB of from 2 to 20 and (b) a melting point equal to, or higher than, the temperature of the slurry at the time of said dehydrating treatment.

The absorbent web structure of this invention obtained by a dry sheet forming process, is composed of a mixture of 5 to 50% by weight of short fibers of a thermoplastic resin rendered hydrophilic by a surface-active agent and 95 to 50% by weight of cellulosic fibers, the thermoplastic short fibers being melt-bonded to impart self-supporting property to the structure, characterized in that the thermoplastic short fibers satisfy the conditions (i) and (ii).

The melting point of the nonionic surface-active agent used in this invention is determined by JIS K-0064.

The thermoplastic short fibers used in this invention may be obtained by melt-spinning a thermoplastic resin, such as an olefin resin derived from one or more α-olefins, for example polyethylene, polypropylene, an ethylene/propylene copolymer, an ethylene/1-butene copolymer or an ethylene/4-methylpentene copolymer, by various methods, and then cutting the resulting filaments. There can also be used split yarns obtained by splitting a film of such a thermoplastic resin as exemplified above, or pulp-like materials (referred to as synthetic pulp) obtained by the flash spinning of the aforesaid thermoplastic resin.

The synthetic pulp is preferred because it has good miscibility with the cellulosic fibers such as wood pulp of the absorbent web structure to provide a uniform mixture. A method for producing synthetic pulp is disclosed, for example, in Japanese Patent Publication No. 47049/1977 cited hereinabove. In the present invention, synthetic pulp treated with polyvinyl alcohol is preferred which is produced by using polyvinyl alcohol in the production of synthetic pulp.

The surface-active agent used in this invention is nonionic, and has an HLB value in the range of 2 to 20. If the HLB value is smaller than 2 or larger than 20, sufficient hydrophilicity cannot be imparted to the thermoplastic short fibers. In order to improve absorbency, therefore, it is essential to use nonionic surface-active agents having an HLB value within the above-specified range as well as to satisfy the melting conditions and hydrophilicity-imparting treating conditions to be described in detail hereinbelow.

Surfactants having an HLB outside the range specified in this invention, such as polyvinyl alcohol, are not used in the absorbency-improving treatment in accordance with this invention. The thermoplastic short fibers used in the hydrophilicity-imparting treatment of this invention in an aqueous slurry may be those which have already been treated with surfactants outside the scope of the nonionic surfactants used in this invention.

It is essential that the melting point (determined by JIS K-0064) of the nonionic surface-active agent used in this invention be equal to, or higher than, the temperature of the aqueous slurry during dehydration in the hydrophilicity-imparting treatment in accordance with this invention.

The synthetic pulp of thermoplastic resin assumes the state of an aqueous slurry of synthetic pulp in the final stage of its production, and is dehydrated. Under manufacturing conditions having good efficiency, the temperature of the aforesaid aqueous slurry is in the range of about 10 to about 50°C. The melting point (JIS K-0064) of the nonionic surface-active agent used in this invention is desirably equal to, or higher than, the temperature of the aqueous slurry during dehydration, and is, for example in the range of about 20 to about 80°C, preferably about 30 to about 80°C, especially preferably at least about 50°C.

Those surface-active agents which have a melting point (JIS K-0064) below the temperature of the aqueous slurry during the dehydrating treatment are liquid in the aqueous slurry, and therefore, their adhesion to the thermoplastic short fibers becomes poor. Consequently, such surface-active agents are liable to escape during the dehydration treatment, and do not easily adhere to the thermoplastic short fibers.

The nonionic surfactant used in this invention meets the above HLB and melting point requirements.

Many surface-active compounds similar to the nonionic surfactants specified in this invention do not come within the range specified in this invention because of differences in molecular weight, degree of polymerization and degree of esterification. Examples of preferred nonionic surfactants used in this invention are shown below, but they must further be screened to conform to the requirements set forth herein.

Polyoxyethylene alkyl ethers, polyoxyethylene alkyl phenyl ethers (e.g., polyoxyethylene nonyl phenyl ether), polyoxyethylene fatty acid esters, sorbitan fatty acid esters (e.g., sorbitan monoleate, sorbitan monopalmitate, sorbitan sesquioleate), polyoxyethylene sorbitan fatty acid esters, and glycerin fatty acid esters (e.g., glycerin monostearate). Especially preferred are glycerin fatty acid esters having an HLB of 2 to 6 and a melting point (JIS K-0064) of 40 to 80°C, sorbitan fatty acid esters having an HLB of 2 to 8 and a melting point (JIS K-0064) of 20 to 80°C, and polyoxyethylene alkyl phenyl ethers having an HLB of 8 to 20 and a melting point (JIS K-0064) of 10 to 50°C.

The thermoplastic short fibers used in this invention are prepared by forming an aqueous slurry of the fibers containing a nonionic surface-active agent meeting the requirements given in this invention, and then dehydrating the aqueous slurry. As necessary, the dehydrated product may be dried. The preferred take-up of the surfactant in the resulting thermoplastic short fibers is about 0.1 to about 5% by weight based on the weight of the fibers.

Examples of the cellulosic fibers, the other component of the absorbent web structure of this invention include various wood pulps and regenerated cellulosic fibers such as acetate fibers and viscose fibers.

The absorbent web structure of this invention is obtained by a dry sheet forming process and is composed of a mixture of 5 to 50% by weight of the thermoplastic resin short fibers treated with a nonionic surfactant as state hereinabove and 95 to 50% by weight of the cellulosic fibers.

The web structure of the invention can be obtained by heating the dry or wet web-like material composed of the above mixture to melt-bond the thermoplastic short fibers.

If the proportion of the thermoplastic short fibers is less than 5% by weight, scarcely any improvement in mechanical strength is obtained by the melt-bonding treatment. If, on the other hand, it exceeds 50% by weight, a reduction in absorbency cannot be avoided.

The melt-bonded treatment of the thermoplastic short fibers can be effected, for example, by using an air oven or an infrared heater. The heating temperature may vary depending upon the type of the thermoplastic resin constituting the thermoplastic short fibers, but is preferably from the melting point of the thermoplastic resin used to a temperature about 50°C higher than it.

The bulk density of the absorbent web structure of this invention can be adjusted to some extent by the melt-bonding treatment of the thermoplastic short fibers. If desired, products of varying bulk densities can be obtained by performing a moderate press treatment simultaneously with the melt-bonding treatment.

The absorbent web structure of this invention may include another water-holding material in order to improve its absorbency further. For example, fine particles of various polymeric electrolytes can be used as such as water-holding material, as disclosed in the above-cited Japanese Laid-Open Patent Publication No. 16611/1980. Preferred water-holding materials include for example, polymers resulting from grafting of a vinyl compound, such as acrylic acid or acrylonitrile, which has a hydrophilic group or a group convertible to a hydrophilic group by hydrolysis to polyglucose or saccharose such as wood pulp, cotton or starch, and hydrolysis products of such graft polymers.

The absorbent web structure of this invention has especially good penetrability of an aqueous liquid (absorbency rate), and excellent mechanical properties such as elasticity and recovery. It further has excellent properties suitable for use as disposable diapers, sanitary napkins, medical sponges wound-treating pads and towels. Depending upon the ultimate uses, an outer covering material having reduced water-holding property or a water-impervious lining material may be laminated to the web structure of this invention.

The following examples illustrate the present invention in greater detail.

Example 1

Twenty grams of synthetic pulp (average fiber length 0.9 mm ) of flash-spun fibers of high-density polyethylene was put in 1 liter of water kept at 40°C, and 150 mg of glycerin monostearate (HLB 3.2; melting point, JIS K-0064, 55°C) was added. The mixture was stirred to form an aqueous slurry. The aqueous slurry (40°C) was dehydrated between wire gauzes until its water content was decreased to 30% by weight, and then dried under heat.

The resulting synthetic pulp had 0.75% by weight of glycerin monostearate adhering to its surface.

Twelve grams of the synthetic pulp and 48g of crushed pulp were uniformly mixed, and subjected to a dry sheet forming process to form a web having a basis weight of 375 g/m². The web was treated in an air oven at 150°C for 10 minutes to melt-bond the fibers of the synthetic pulp.

In accordance with No. 33-80 "Determination of Water Absorbency Rate of Bibulous Paper (Water Drop Method)" in J. TAPPI Testing Methods for Paper and Pulp, the resulting absorbent web structure was laid horizontally. One cubic centimeter of tap water at 20±2°C was added dropwise by means of a syringe. The time required for the water droplets to completely penetrate into the inside of the test sample from its surface was measured. It was 0.7 second.

Examples 2 to 6 and comparative examples 1 to 7

Twenty grams of cut fibers of polypropylene (3 denier×5mm; P-Chop, a trade name for a product of Chisso Co., Ltd.) were put in 1 liter of water kept at 23°C, and 200 mg of each of the surface-active agents indicated in Table 1 was added. The mixture was stirred to form an aqueous slurry. The slurry (23°C) was dehydrated between wire gauzes until its water content was decreased to 30% by weight, and then dried under heat.

Using the treated cut fibers, a web was produced in the same way as in Example 1. The web was treated in an air oven at each of the temperatures shown in Table 1 for 5 minutes to melt-bond the cut fibers.

The products were tested as in Example 1 for hydrophilic properties, and the results are shown in Table 1.

TABLE 1

| Run | Surface-active agent | | | | | Take-up (wt.%) | Time required for water absorption (seconds) | |
| | Type | Form | Melting point (°C) (JIS K-0064) | HLB | | | After melt-bonding treatment | |
| | | | | | | | at 140°C, 5 min. | at 150°C, 5 min. |
|---|---|---|---|---|---|---|---|---|
| Ex. 2 | Sorbitan monopalmitate (Nonion PP4 OR) | Solid | 48±3 | 6.7 | | 0.92 | 0 | 0 |
| Ex. 3 | Stearyl monoglyceride (Atmos 150) | Solid | 55±5 | 3.2 | | 0.70 | 0 | 0 |
| Ex. 4 | POE nonylphenyl ether (Nonion NS220) | Semi-solid | 27±3 | 16.0 | | 0.99 | 2 | 7 |
| Ex. 5 | Ditto (Nonion NS230) | Solid | 38±3 | 17.1 | | 0.80 | 3 | 4 |
| Ex. 6 | Ditto (Emulgen 950) | Solid | 33±3 | 18.2 | | 0.82 | 5 | 13 |
| CEx. 1 | Sorbitan sesquioleate (Solgen 30) | Liquid | 1±2 | 3.7 | | 0.10 | 27 | 300< |
| CEx. 2 | Ditto (Solgen 40) | Liquid | −3±2 | 4.3 | | 0.05 | 20 | 300< |
| CEx. 3 | Sorbitan monolaurate (Nonion LP-20R) | Liquid | 10±3 | 8.6 | | 0.20 | 300< | 300< |
| CEx. 4 | POE lauryl ether (Emulgen 108) | Liquid | 10±3 | 12.8 | | 0.28 | 300< | 300< |
| CEx. 5 | POE nonyl phenyl ether (Nonion NS212) | Liquid | 12±3 | 13.3 | | 0.15 | 27 | 300< |
| CEx. 6 | Sorbitan trioleate | Semi-solid | 30±3 | 1.8 | | 0.65 | 30 | 300< |
| CEx. 7 | Sodium laurylsulfate | Liquid | 20±3 | 40 | | 0.02 | 300< | 300< |

Note: Ex.=Example,  CEx.=Comparative Example.

## Claims

1. An absorbent web structure obtained by a dry sheet forming process composed of a mixture of 5 to 50% by weight of short fibers of a thermoplastic resin rendered hydrophilic with a surface-active agent and 95 to 50% by weight of cellulosic fibers, said thermoplastic short fibers being melt-bonded to impart self-supporting property to the web structure; characterized in that

(i) said thermoplastic short fibers have been rendered hydrophilic by first forming an aqueous slurry of the fibers containing a nonionic surface-active agent and then dehydrating the slurry, and

(ii) said nonionic surface-active agent has (a) an HLB value of from 2 to 20 and (b) a melting point equal to, or higher than, the temperature of the slurry at the time of said dehydrating treatment.

2. A structure according to claim 1 wherein a synthetic pulp composed of flash-spun fibers of the thermoplastic resin is used in (i).

3. A structure according to claim 2 wherein the synthetic pulp is a synthetic pulp treated with polyvinyl alcohol.

4. A structure according to claim 1, 2 or 3 wherein the temperature of the slurry at the time of said dehydrating treatment is not more than 50°C.

5. A structure according to any one of the preceding claims wherein the take-up of the nonionic surface-active agent in the thermoplastic short fibers is 0.1 to 5% by weight based on the weight of the fibers.

6. A structure according to any one of the preceding claims wherein the nonionic surface-active agent has a melting point of at least about 50°C.

7. A structure according to any one of the preceding claims wherein the thermoplastic resin is an olefin resin.

8. A structure according to any one of the preceding claims wherein the melt-bonding temperature is from the melting point of the thermoplastic resin to a temperature about 50°C higher than it.

## Patentansprüche

1. Saugfähiges Vliesmaterial, erhalten durch ein Trockenformverfahren für Folien, bestehend aus einem Gemisch aus 5 bis 50 Gew.-% Kurzfasern eines thermoplastischen Harzes, das mit einem oberflächenaktiven Mittel hydrophil gemacht worden ist, und aus 95 bis 50 Gew.-% cellulosischen Fasern, wobei die thermoplastischen Kurzfasern schmelz-gebunden sind, um dem Vliesmaterial selbsttragende Eigenschaft zu verleihen, dadurch gekennzeichnet, daß

(i) die thermoplastischen Kurzfasern hydrophil gemacht worden sind, indem zunächst eine wäßrige Aufschlämmung der Fasern, enthaltend ein nichtionisches oberflächenaktives Mittel, hergestellt und dann getrocknet worden ist, und

(ii) das nichtionische oberflächenaktive Mittel (a) einen HLB-Wert von 2 bis 20 und (b) einen Schmelzpunkt aufweist, der gleich oder höher ist als die Temperatur der Aufschlämmung zur Zeit der Trocknungsbehandlung.

2. Material nach Anspruch 1, bei dem ein synthetischer Stoffbrei, zusammengesetzt aus schnellgesponnenen Fasern des thermoplastischen Harzes, in (i) verwendet worden ist.

3. Material nach Anspruch 2, bei dem der synthetische Stoffbrei ein mit Polyvinylalkohol behandelter synthetischer Stoffbrei ist.

4. Material nach Anspruch 1, 2 oder 3, bei dem die Temperatur der Aufschlämmung zur Zeit der Trocknungsbehandlung nicht mehr als 50°C betragen hat.

5. Material nach einem der vorangehenden Ansprüche, bei dem die Aufnahme des nichtionischen oberflächenaktiven Mittels in die thermoplastischen Kurzfasern 0,1 bis 5 Gew.-% ausmacht, bezogen auf das Gewicht der Fasern.

6. Material nach einem der vorangehenden Ansprüche, bei dem das nichtionische oberflächenaktive Mittel einen Schmelzpunkt von nicht weniger als etwa 50°C aufweist.

7. Material nach einem der vorangehenden Ansprüche, bei dem das thermoplastische Harz ein Olefinharz ist.

8. Material nach einem der vorangehenden Ansprüche, bei dem die schmelz-bindende Temperatur vom Schmelzpunkt des thermoplastischen Harzes bis zu einer Temperatur etwa 50°C und darüber reicht.

## Revendications

1. Structure de bande absorbante obtenue par un procédé de formage de feuilles à sec, composée d'un mélange de 5 à 50 % en poids de fibres courtes en résine thermoplastique, rendues hydrophiles par un agent tensioactif, et 95 à 50 % en poids de fibres cellulosiques, lesdites fibres thermoplastiques courtes étant liées par fusion afin de conférer une propriété de cohésion à la structure, caractérisée en ce que:

(i) lesdites fibres thermoplastiques courtes sont rendues hydrophiles par formation d'une bouillie aqueuse des fibres qui contient un agent tensioactif, et déshydratation ultérieure de la bouillie, et

(ii) ledit agent tensio-actif non ionique présente (a) un rapport hydro-lipophile (RHL) de 2 à 20 et (b) un point de fusion égal ou supérieur à la température de la bouillie au moment du traitement de déshydratation décrit dans (i) ci-dessus.

2. Structure selon la revendication 1, dans laquelle on utilise dans (i) une pâte synthétique composée de fibres filées par détente d'une résine thermoplastique.

3. Structure selon la revendication 2, dans laquelle la pâte synthétique est une pâte synthétique traitée avec du poly(alcool vinylique).

4. Structure selon la revendication 1, 2 ou 3, dans laquelle la température de la bouillie pendant ledit traitement de déshydratation n'est pas supérieure à 50°C.

5. Structure selon l'une quelconque des revendications précédentes, dans laquelle la quantité de l'agent tensioactif non ionique absorbée dans les fibres thermoplastiques courtes est de 0,1 à 5% en poids par rapport au poids des fibres.

6. Structure selon l'une quelconque des revendications précédentes, dans laquelle l'agent tension-actif non ionique présente un point de fusion d'au moins 50°C environ.

7. Structure selon l'une quelconque des revendications précédentes, dans laquelle la résine thermoplastique est une résine d'oléfine.

8. Structure selon l'une quelconque des revendications précédentes, dans laquelle la température de liaison par fusion est comprise entre le point de fusion de la résine thermoplastique et une température supérieure d'environ 50°C à ce point de fusion.